# EUROPÄISCHE PATENTSCHRIFT

(11) **EP 3 721 841 B1**
(45) Veröffentlichungstag und Bekanntmachung des Hinweises auf die Patenterteilung: **03.12.2025**
(21) Anmeldenummer: 20000136.0
(22) Anmeldetag: 30.03.2020
(51) Int. Cl.: A61F 5/11

(54) **VERBINDUNGSELEMENT FÜR MEHRTEILIGE NAGELKORREKTURSPANGEN**
CONNECTING ELEMENT FOR A MULTI-PIECE NAIL CORRECTION CLIP
ÉLÉMENT DE RACCORDEMENT POUR ORTHÈSES UNGUÉALES EN PLUSIEURS PIÈCES

(30) Priorität: 09.04.2019 DE 102019002568
(43) Veröffentlichungstag der Anmeldung: 14.10.2020
(73) Patentinhaber: 3TO GmbH, 82941 Deisenhofen (DE)
(72) Erfinder: Sutor, Johannes, 80469 München (DE)
(74) Vertreter: Müller, Bernhard

(56) Entgegenhaltungen:
- EP-A1- 2 508 154
- WO-A1-2012/017527
- DE-A1- 3 711 755
- DE-C2- 3 711 755
- DE-U1- 202006 009 739
- KR-A- 20150 137 289

## Beschreibung

Die Erfindung betrifft ein Verbindungselement für mehrteilige Nagelkorrekturspangen zur Korrektur von menschlichen Nägeln, insbesondere von Zehennägeln, gemäß Oberbegriff des Hauptanspruchs.

Zur Behandlung von eingewachsenen oder eingerollten Nägeln, insbesondere Großzehennägeln, wurden als Alternative zur schmerzhaften und mit langen Genesungszeiten verbundenen Operation (Emmert-Plastik) verschiedene Nagelkorrekturspangen entwickelt. Diese sogenannten Orthonyxiespangen reduzieren die Krümmung der Nagelplatte und entlasten dadurch den oftmals entzündeten Nagelfalz. Die Schmerzen werden deutlich gemindert und eine Abheilung ermöglicht.

Die Wirkung dieser Nagelkorrekturspangen beruht auf Rückstellkräften, außermittigen Zugkräften oder auf Kombinationen dieser beiden Mechanismen. In der Praxis sind einteilige und mehrteilige Spangensysteme bekannt. Bei einigen mehrteiligen Systemen wird die Kraft der Nagelkorrekturspange über ein Verbindungselement aufgebracht, welches den Abstand zwischen zwei Krafteinleitungselementen verringert und somit ein Biegemoment erzeugt, um die Nagelplatte an ihren Rändern leicht anzuheben.

Folgende mehrteilige Vorrichtungen zur Nagelkorrektur mit entsprechendem Verbindungselement sind bisher bekannt und werden zur Behandlung eingesetzt. Die beschriebenen Verbindungselemente weisen jedoch entweder bezüglich ihrer Wirksamkeit oder ihrer Anwendung einige Nachteile auf: WO2012017527 ist der nächstliegende Stand der Technik und offenbart eine Vorrichtung zur Korrektur eines deformierten Nagels, z. B. eines eingewachsenen Nagels, mit Verriegelungselementen.

### Nagelkorrekturspange, bestehend aus 2 Ankerplättchen mit einem losen Zugelement

Eine Vorrichtung, bestehend aus zwei, auf den Nagel aufgeklebten Ankern, welche durch ein loses Zugelement miteinander verbunden werden, ist aus der DE 32 33 419 A1 bekannt. Hier können die Elemente zur Krafteinleitung im ungespannten Zustand auf die Nagelplatte geklebt werden. Die Einstellung der Spangenkraft erfolgt über ein elastisches Zugelement, wie zum Beispiel einen Gummiring, wobei die Intensität der Spangenkraft vom Abstand der Befestigungselemente und der Größe und Stärke des Zugelements abhängt und daher nicht genau und nicht stufenlos einstellbar ist.

Die Dosierung der Kraft ist, bedingt durch die vorgegebenen Gummilängen und -stärken sowie die variablen Abstände zwischen den Krafteinleitungselementen, nicht stufenlos möglich, zudem kann das nur lose befestigte Zugelement verloren werden.

### Drahtspange aus 3 Teilen (2 Schenkel + 1 Schlaufe; DE 42 07 797 A1)

Eines der am weitesten verbreiteten und trotz diverser neuerer Entwicklungen noch immer sehr häufig eingesetzten Systeme am Markt stellt die dreiteilige Drahtspange dar, welche aus zwei mit Häkchen versehenen Schenkeln und einer in der Mitte anzubringenden Drahtschlaufe besteht. Der Therapeut versieht die teilweise vorgefertigten Spangenschenkel mit Häkchen (oder passt diese im Falle einer industriellen Vorfertigung derselben an die Nageldicke an) und formt die Spange entsprechend der Nagelkrümmung. Als Krafteinleitungselemente werden die Schenkel einzeln unter den Nagelrand eingehängt und mit der vorgefertigten Schlaufe verbunden, wodurch die Spangenbreite an die Nagelbreite angepasst und die Spangenkraft stufenlos in Absprache mit dem Patienten eingestellt werden kann.

Die Anwendung dieser Spangen erfordert einiges Geschick des Therapeuten.

Auf Grund der geringen Abmessungen ist besonders das Anbringen der Mittelschlaufe sehr aufwändig und führt oftmals wieder zum Herausfallen der seitlichen Spangenschenkel. Zudem besteht die Gefahr, dass die Mittelschlaufe reißt, wenn sie nicht absolut zentrisch zusammengedreht oder wieder zurück gedreht wird, da die vorgefertigten Ösen abbrechen.

Aufgabe der vorliegenden Erfindung ist es, eine Vorrichtung zur Nagelkorrektur zu schaffen, welche ohne besondere Fingerfertigkeit anwendbar ist, und mit der die erforderlichen Spangenkräfte in einfacher Weise stufenlos eingestellt werden können.

Im Laufe von Entwicklungsarbeiten im Rahmen der vorliegenden Erfindung wurde festgestellt, dass es von besonderem Vorteil ist, das Verbindungselement für mehrteilige Nagelkorrekturspangen so zu gestalten, dass durch Einwirkung einer stufenlos verstellbaren, definierbaren Kraft ein Biegemoment auf die Nagelplatte übertragen wird. Dabei hat es sich als besonders vorteilhaft erwiesen, wenn das Verbindungselement als geschlossener Ring ausgeführt ist. Das Verbindungselement mit geschlossener Kontur kann einfach zwischen die Krafteinleitungselemente eingehängt werden und auf diese durch das Verkürzen des Verbindungselementes eine stufenlos verstellbare Zugkraft ausüben.

Der Gegenstand der Erfindung ist in Anspruch 1 definiert.

Fortentwicklungen und besondere Ausführungsformen der Erfindung ergeben sich aus den Unteransprüchen.

Nachstehend wird die Erfindung beispielhaft anhand einiger Zeichnungen näher erläutert:
Dabei zeigen:
Fig. 1 das erfindungsgemäße Verbindungselement für Nagelkorrekturspangen in seiner bevorzugten Ausführung;
Figg. 2 bis 5 das Aktivieren einer dreiteiligen Nagelkorrekturspange aus Draht durch Verdrillen des erfindungsgemäßen Verbindungselementes;
Fig. 6 das erfindungsgemäße Verbindungselement im Zustand vor dem Spannen, angebracht an einem Nagelkorrekturspangensystem mit aufklebbaren Krafteinleitungselementen.

Nachstehend wird auf die in den Figuren dargestellten Ausführungsformen näher eingegangen.

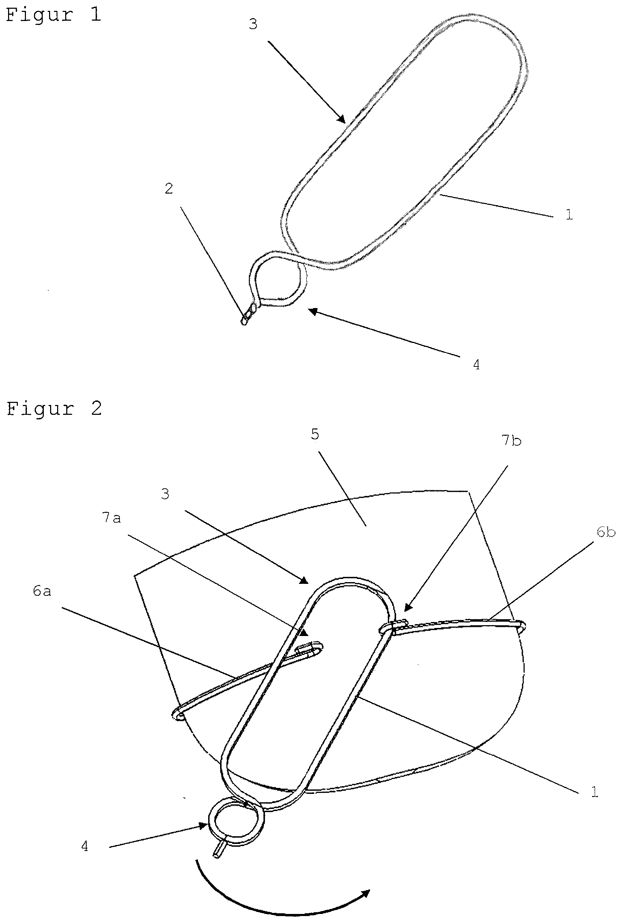

Figur 1 zeigt das erfindungsgemäße Verbindungselement (1) für Nagelkorrekturspangen in seiner bevorzugten Ausführung als geschlossener Ring aus Draht, welcher durch eine Verdrehung um 180° um die Mittelachse in Form einer Acht ausgebildet ist. Als bevorzugter Werkstoff wird schlussgeglühter Edelstahldraht im Durchmesserbereich zwischen 0,2 mm und 0,5 mm verwendet, welcher eine hohe plastische Verformbarkeit bei gleichzeitig hoher Zugfestigkeit aufweist. Für die Ausbildung der geschlossenen Kontur können die Enden eines Drahtstückes mittels Formschluss, wie beispielsweise durch eine Umschlingung (2) oder durch Stoffschluss, wie beispielsweise durch eine stumpfe oder parallele Verschweißung fest miteinander verbunden werden. Alternativ kann die geschlossene Kontur durch Ausstanzen eines filigranen Ringes aus einem Blech hergestellt werden.

Die längere, untere Öse (3) dient der Verbindung der Krafteinleitungselemente der Nagelkorrekturspange, die kleinere, obere Öse (4) dient der Aufnahme eines Hakens zum Verdrillen des Verbindungselementes (1). Durch die ovale, seitlich abgeflachte Ausprägung der unteren Öse (3) kann das Verbindungselement (1) besonders einfach in die Krafteinleitungselemente der Nagelkorrekturspange eingehängt werden.

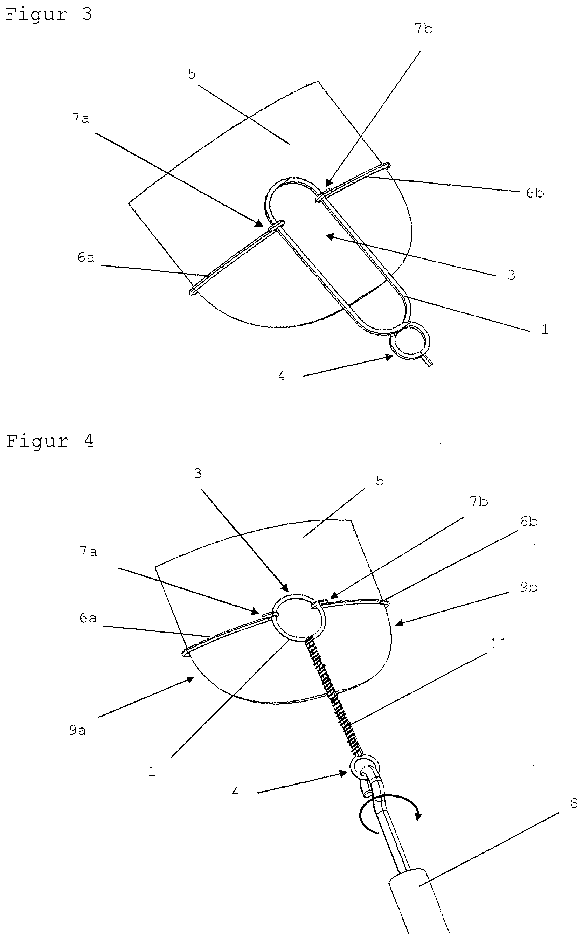

So kann, wie in Figur 2 dargestellt, das Verbindungselement (1) zunächst fast quer zur Nagellängsachse zwischen die unter den Nagel (5) eingehängten Krafteinleitungselemente (6a, 6b) eingelegt werden. Anschließend wird das Verbindungselement (1) zur Mittelachse nach vorne gedreht und umgreift dadurch, wie in Figur 3 gezeigt, die Haken (7a, 7b) der Krafteinleitungselemente (6a, 6b).

Zur Behandlung des eingewachsenen Nagels (5) sollen die Nagelränder minimal angehoben werden, um den Nagelfalz zu entlasten. Hierzu wird, wie in Figur 4 dargestellt, das Verbindungselement (1) mittels eines in die obere Öse (4) eingehängten Windehakens (8) verdrillt. Durch das Verdrillen wird die zwischen den Haken (7a, 7b) der Krafteinleitungselemente (6a, 6b) eingehängte Öse (3) des Verbindungselementes (1) verkleinert, der Abstand zwischen den Krafteinleitungselementen (6a, 6b) wird verringert. Durch den hierdurch entstehenden, außermittigen Zug wird ein Biegemoment auf den Nagel (5) aufgebracht, die Nagelränder (9a, 9b) werden angehoben.

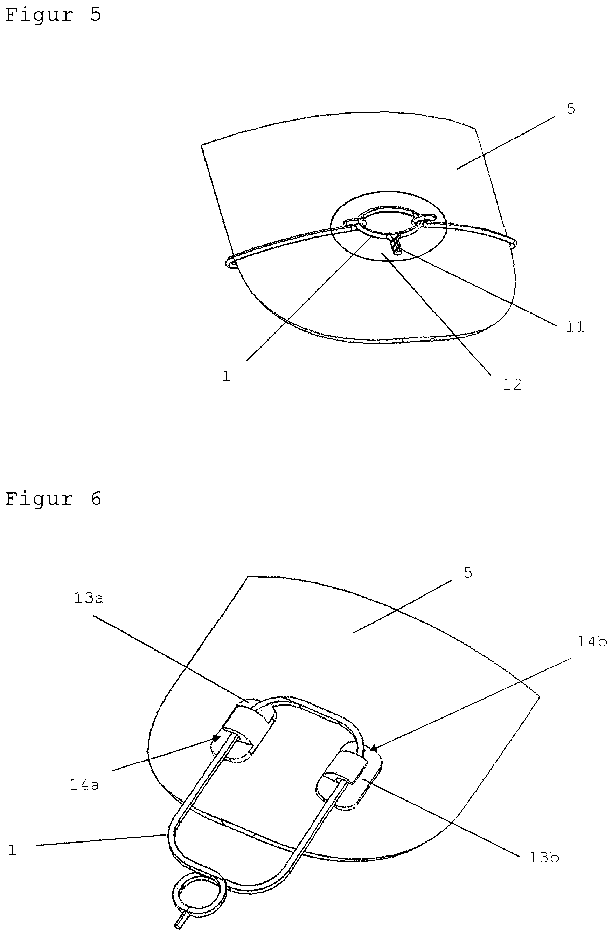

Figur 5 zeigt die fertig angebrachte Nagelkorrekturspange. Die entstandene Spirale (11) des Verbindungselementes (1) wird auf zwei bis drei Windungen gekürzt. Um die Verbindung zu fixieren und den Patienten vor Verletzungen zu schützen, wird das Verbindungselement (1) mit einer Schutzschicht (12) aus aushärtender Kunststoffmasse überzogen.

Figur 6 zeigt das erfindungsgemäße Verbindungselement (1) an einer Nagelkorrekturspange mit auf den Nagel (5) aufgeklebten Krafteinleitungselementen (13a, 13b). Die Krafteinleitungselemente (13a, 13b) sind meist aus Kunststoff gefertigt und weisen jeweils eine Klebefläche, welche mittels eines schnell aushärtenden Klebstoffes auf dem Nagel (5) fixiert werden kann, sowie je einen Haken (14a, 14b) auf. In die Haken (14a, 14b) wird das Verbindungselement (1) eingehängt und kann anschließend durch Verdrillung gespannt werden. Diese Variante ermöglicht das Aufbringen eines stufenlos einstellbaren Biegemomentes auf die Nagelplatte, ohne dass Haken unter den Nagelrand eingehängt werden müssen.

### Vorteile der erfindungsgemäßen Vorrichtung zur Nagelkorrektur

Gegenüber den bisher bekannten, herkömmlichen Verfahren und Vorrichtungen weist das erfindungsgemäße Verbindungselement für mehrteilige Nagelkorrekturspangen einige Vorteile auf.

Durch seine geschlossene Kontur werden die Anwendung des Verbindungselementes und damit die Anwendung von mehrteiligen Nagelkorrekturspangen im Gesamten deutlich vereinfacht. Das Herausspringen der seitlichen Spangenschenkel bei dreiteiligen Nagelkorrekturspangen aus Draht beim Einfädeln des Verbindungselementes zwischen die Spangenschenkel, wie es bei nicht geschlossener Bauform des Verbindungselementes regelmäßig auftritt, wird hierdurch vermieden. Auch das Einhängen des Windewerkzeuges zum Verdrillen ist beim erfindungsgemäßen Verbindungselement mit einer geschlossenen Öse deutlich einfacher als bei der bekannten Ausführung mit zwei Ösen an zwei losen Enden.

Die bisher bekannten Verbindungselemente aus Draht haben den Nachteil, dass die ausgebildeten Ösen bei einem nicht vorschriftsmäßigen Zusammendrehen entweder aufgehen oder abbrechen können. Durch die Einstückigkeit und die damit verbundene gleichmäßige Materialstärke des erfindungsgemäßen Verbindungselementes in seiner Ausführung als geschlossener Drahtring ist die Gefahr des Reißens beim Verdrillen deutlich minimiert, ein Aufdrehen kann nicht stattfinden.

Gegenüber einem elastischen Gummiring als Verbindungselement zwischen zwei auf den Nagel aufgeklebten Krafteinleitungselementen weist das erfindungsgemäße Verbindungselement ebenfalls deutliche Vorteile auf. So kann die Zugkraft stufenlos und reversibel aufgebracht werden. Die Gefahr des Verlierens des Verbindungselementes ist durch die nach dem Spannen starre Verbindung nicht mehr gegeben.

Durch die genannten Vorteile wird die Anwendung mehrteiliger Nagelkorrekturspangen durch den Einsatz des erfindungsgemäßen Verbindungselementes deutlich erleichtert und wird dadurch der Anforderung der Wirtschaftlichkeit in der ärztlichen, podologischen und kosmetischen Praxis besonders gerecht.

## Patentansprüche

1. Vorrichtung zur Korrektur von menschlichen Nägeln, die zwei hakenförmige Krafteinleitungselemente (6a, 6b), die unter den Rand eines Nagels einhängbar oder auf den Nagel aufklebbar sind, und ein in die Krafteinleitungselemente einhängbares Verbindungselement(1) aus flexiblem Material aufweist, wobei das Verbindungselement so verkürzt werden kann, dass auf die Krafteinleitungselemente (6a, 6b) eine stufenlos einstellbare Zugkraft wirkt, die durch außermittigen Kraftangriff ein Biegemoment auf die Nagelplatte überträgt, wobei das Verbindungselement in Form einer Acht mit einer längeren, unteren Öse (3) und einer kleineren, oberen Öse vorliegt, wobei die untere Öse (3) eine ovale, seitlich abgeflachte Form aufweist, die durch Verdrillen verkürzt werden kann, und die obere Öse (4) zur Aufnahme eines Hakens zum Verdrillen des Verbindungselements (1) dient, wodurch das Verbindungselement (1) formschlüssig in der erreichten Position bleibt,
**dadurch gekennzeichnet, dass**
das Verbindungselement als einstückige Doppelschlinge aus schlussgeglühtem, weichen Draht vorliegt.

2. Vorrichtung nach Anspruch 1, **dadurch gekennzeichnet, dass** das Verbindungselement durch enges Verdrillen der losen Enden eines Drahtstückes hergestellt worden ist.

3. Vorrichtung nach Anspruch 1, **dadurch gekennzeichnet, dass** das Verbindungselement durch Verschweißen der losen Enden eines Drahtstücks hergestellt worden ist.

## Claims

1. Device for correcting human nails, comprising two hook-shaped force introduction elements (6a, 6b) that can be hooked under the edge of a nail or stuck onto the nail, and a connecting element (1) made of flexible material that can be hooked into the force introduction elements, wherein the connecting element can be shortened so that a continuously adjustable tensile force acts on the force introduction elements (6a, 6b), wherein the tensile force transmits a bending moment to the nail plate by means of an off-centre force application, wherein the connecting element is in the form of a figure eight with a longer lower eyelet (3) and a smaller upper eyelet (4), wherein the lower eyelet (3) has an oval, laterally flattened shape which can be shortened by twisting, and the upper eyelet (4) serves to accommodate a hook for twisting the connecting element (1), whereby the connecting element (1) remains in a form-fitting manner in the position reached, **characterized in that** the connecting element is a single-piece double loop made of soft wire that has been annealed.

2. Device according to claim 1, **characterized in that** the connecting element has been produced by tightly twisting the loose ends of a piece of wire.

3. Device according to claim 1, **characterized in that** the connecting e
lement has been produced by welding the loose ends of a piece of wire.

## Revendications

1. Dispositif pour corriger les ongles humains, comprenant deux éléments d'introduction de force en forme de crochet (6a, 6b) pouvant être accrochés sous le bord d'un ongle ou collés sur l'ongle, et un élément de liaison (1) en matériau flexible pouvant être accroché dans les éléments d'introduction de force, l'élément de liaison pouvant être raccourci de telle sorte qu'une force de traction réglable en continu agisse sur les éléments d'introduction de force (6a, 6b), transmettant ainsi un moment de flexion à la plaque de l'ongle par application d'une force décentrée, l'élément de liaison se présentant sous la forme d'un huit avec un œillet inférieur plus long (3) et un œillet supérieur plus petit (4), l'œillet inférieur (3) ayant une forme ovale aplatie latéralement qui peut être raccourcie par torsion, et l'œillet supérieur (4) sert à recevoir un crochet pour tordre l'élément de liaison (1), de sorte que l'élément de liaison (1) reste dans la position atteinte par complémentarité de forme, **caractérisé en ce que** l'élément de liaison se présente sous la forme d'une double boucle monobloc en fil métallique doux recuit.

2. Dispositif selon la revendication 1, **caractérisé en ce que** l'élément de liaison a été fabriqué en tordant étroitement les extrémités libres d'un morceau de fil métallique.

3. Dispositif selon la revendication 1, **caractérisé en ce que** l'élément de liaison a été fabriqué en soudant les extrémités libres d'un morceau de fil métallique.
